# EUROPEAN PATENT SPECIFICATION

(11) **EP 2 067 489 B1**
(45) Date of publication and mention of the grant of the patent: **23.07.2014**
(21) Application number: 07122317.6
(22) Date of filing: 04.12.2007
(51) Int. Cl.: A61K 49/00, A61K 51/00, G01N 33/66

(54) **Glyco-molecular imaging method for grade classification of liver fibrosis and its glyco-molecular imaging agent thereof**
Glykomolekulares Abbildungsverfahren zur Einstufung des Leberfibrosegrades und glykomolekulares Abbildungsmittel dafür
Procédé d'imagerie glyco-moléculaire pour la classification du niveau de fibrose du foie et agent d'imagerie glyco-moléculaire correspondant

(43) Date of publication of application: 10.06.2009
(73) Proprietor: Institute of Nuclear Energy Research Atomic Energy Council, Longtan Township, Taoyuan County (TW)
(72) Inventor: Wang, Mei-Hui c/o Inst. of Nucl. Energy Research, Longtan Township, Taoyuan County (TW); Lin, Li-Fu c/o Inst. of Nucl. Energy Research, Longtan Township, Taoyuan County (TW); Chen, Haw-Jan c/o Inst. of Nucl. Energy Research, Longtan Township, Taoyuan County (TW); Shen, Lie-Hang c/o Inst. of Nucl. Energy Research, Longtan Township, Taoyuan County (TW); Lee, Shui-Cheng c/o Inst. of Nucl. Energy Research, Longtan Township, Taoyuan County (TW); Lin, Wuu-Jyh c/o Inst. of Nucl. Energy Research, Longtan Township, Taoyuan County (TW)
(74) Representative: Lang, Christian

(56) References cited:
- US-A- 4 401 647
- US-A- 5 118 798
- US-A- 5 679 323
- SHIOMI S ET AL: "Use of scintigraphy with 99mtechnetium galactosyl human serum albumin for staging of primary biliary cirrhosis and assessment of prognosis." JOURNAL OF GASTROENTEROLOGY AND HEPATOLOGY JUN 1999, vol. 14, no. 6, June 1999 (1999-06), pages 566-571, XP002479341 ISSN: 0815-9319
- SASAKI N ET AL: "Clinical usefulness of scintigraphy with 99mTc-galactosyl-human serum albumin for prognosis of cirrhosis of the liver." JOURNAL OF NUCLEAR MEDICINE : OFFICIAL PUBLICATION, SOCIETY OF NUCLEAR MEDICINE OCT 1999, vol. 40, no. 10, October 1999 (1999-10), pages 1652-1656, XP009099790 ISSN: 0161-5505
- I-CHUNG TANG, TSAI-YUEH LUO, CHANG-MAU SHING, CHENG-HSIEN LIN, YUEN-HAN YEH, WUU-JYH LIN, YU-LING WU AND SHAN-YUN CHENG: "Synthesis and characterization of 188Re-SOCTA-Herceptin" J NUCL MED., vol. 48, no. 2, 2007, page 455P, XP009099797
- BROWN J J ET AL: "Imaging of hepatic cirrhosis" RADIOLOGY, OAK BROOK,IL, vol. 202, no. 1, 1 January 1997 (1997-01-01), pages 1-16, XP009099760 ISSN: 0033-8419
- KIM EUN-MI ET AL: "Asialoglycoprotein-receptor-targeted hepatocyte imaging using Tc-99m galactosylated chitosan" NUCLEAR MEDICINE AND BIOLOGY, vol. 33, no. 4, May 2006 (2006-05), pages 529-534, XP002479342 ISSN: 0969-8051
- AKAKI SHIRO ET AL: "Technetium-99m-DTPA-galactosyl human serum albumin liver scintigraphy evaluation of regional CT/MRI attenuation/signal intensity differences" JOURNAL OF NUCLEAR MEDICINE, vol. 39, no. 3, March 1998 (1998-03), pages 529-532, XP009099771 ISSN: 0161-5505
- LUYT L G ET AL: "An N2S2 bifunctional chelator for technetium-99m and rhenium: complexation, conjugation, and epimerization to a single isomer" BIOCONJUGATE CHEMISTRY, ACS, WASHINGTON, DC, US, vol. 10, no. 3, 1 May 1999 (1999-05-01), pages 470-479, XP002468131 ISSN: 1043-1802

## Description

### FIELD OF THE INVENTION

The present invention relates to a molecular imaging method for quantification of liver fibrosis grade and its glyco-imaging agent with liver-targeting characteristics, wherein the agent combining with molecular imaging method could be used to differentiate the grade of liver fibrosis.

### BACKGROUND OF THE INVENTION

In clinics, the liver fibrosis staging often depends on liver biopsy and Metavir classification, which is classified F0, F1, F2, F3, F4 according to the biopsy observation under microscopy. The biggest challenge for this method was its abhorrence by people for its latent danger. Besides, it is not accurate due to the frequent sampling error and deviation of individual observation. Furthermore, the result is qualitative and subjective but not quantitative and objective.

It has been well documented by Lee laboratory since 1983 that multivalent galactopeptide has a strong "cluster effect" to enhance binding with hepatic Gal/GalNAc receptor. The Gal-terminated triantennary structure exhibits 10⁶ fold stronger affinity than monovalent component structure. After binding, the galactopeptide will be endocytosis into hepatic cell. In 2004, Kwon's experiment indicates that the ischemic hepatic membrane has much lower Gal/GalNAc receptor and meanwhile, the endocytosis is also much less than the normal liver cell.

US 5,118,798 discloses a neogalactoalbumin or galactose-bonded polylysine bound to a chelate forming compound such as DTPA which in turn is bound to a radioactive metallic element for use as a radioactive diagnostic or therapeutic agent for liver. US 5,679,323 discloses an MRI contrast agent which is internalized by hepatocytes for obtaining an MR image of the liver to provide a direct measure of liver function and regional difference in the state of the liver. SHIOMI S. et al in "Use of scintigraphy with 99m technetium galactosyl human serum albumin for staging of primary biliary cirrhosis and assessment of prognosis", Journal of Gastroenterology and Hepatology, June 1999, p. 566-571 and SASAKI N. et al in "Clinical usefulness of scinitigraphy with 99mTc-galactosyl serum albumin for prognosis of cirrhosis of the liver", Journal of Nuclear Medicine: Official Publication, Society of Nuclear Medicine, Oct. 1999, p. 1652-1656 disclose use of scintigraphy with 99mTc-DTPA-galactosyl-human serum albumin for staging liver disease and prognosis. US 4,401,647 discloses a galactosyl serum albumin-Tc-99m conjugate having from about 5 to 40 galactosyl groups for scintigraphic hepatic imaging and for use in a method for diagnosing liver disease states.

This invention discloses a glyco-molecular imaging agent with liver-targeting characteristics and a method for synthesizing the same. This glyco-molecular agent allows to provide an exact image distribution and real hepatic cellular uptake value during molecular imaging, which objectively overcomes the numerous uncertainty of prior traditional clinic method.

### SUMMARY OF THE INVENTION

It is therefore an object of the present invention to provide a galactopeptide-containing agent for in vivo accurate grade classification of liver fibrosis, the use of which avoids liver biopsy or body fluid sampling, and allows, when injected, reading the total image signal per unit volume of liver within specified hours.

It is therefore another object of the present invention to supply a glyco-molecular imaging agent for accurate quantification of the liver fibrosis grades, which is made by a bifunctional chelator both linking to galactopeptide and chelating with a metal, and a method for synthesizing the same. Particularly, this bifunctional chelator has highly superior air stability than other bifunctional chelator. The storage time in room temperature is more than one year.

The above-mentioned objects are solved by the galactopeptide-containing agent for in vivo accurate grade classification of liver fibrosis according to claim 1, the method for synthesizing glyco-molecular imaging agent for grade classification of liver fibrosis according to claim 4 and the glyco-molecular imaging agent for grade classification of liver fibrosis according to claim 6. Advantageous improvements of the invention are described by dependent claims.

### BRIEF DESCRIPTION OF THE DRAWINGS

The drawings, incorporated into and form a part of the disclosure, illustrate the embodiments and method related to this invention and will assist in explaining the detail of the invention.

Fig 1 is a graph showing the clinical examination steps for differentiate liver fibrosis grades.

Fig 2 is a graph showing the concept of glyco-molecular imaging for liver targeting.

Fig 3 is a graph showing the flow chart of preparation of Tc-99m-SOCTA-galactopeptide.

Fig 4 is a graph showing the structure of SOCTA.

Fig 5 is a graph showing mass spectrum of serum biomarkers with SELDI-TOF analysis.

Fig 6 is a graph showing statistics of serum biomarkers with SELDI-TOF analysis.

Fig 7 is a graph showing the differential expression of 15.1kD molecular weight protein in normal rat liver and fibrotic rat liver.

Fig 8 is a graph showing the differential expression of 15.9kD molecular weight protein in normal rat liver and fibrotic rat liver.

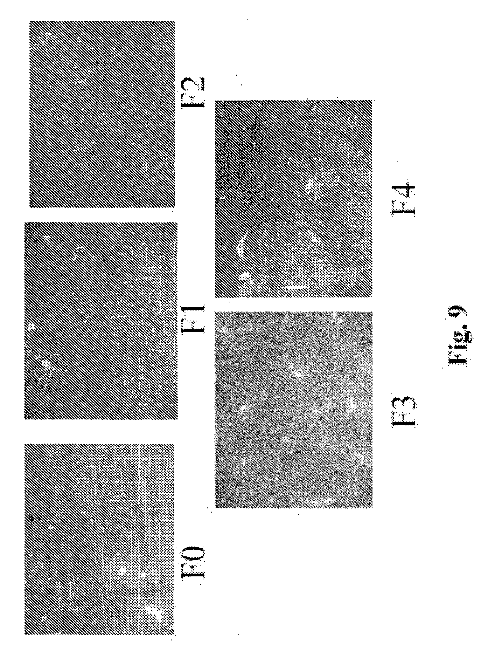

Fig 9 is a graph showing the present grading result of fibrotic liver by pathological examination.

### DESCRIPTION OF THE PREFERRED EMBODIMENT

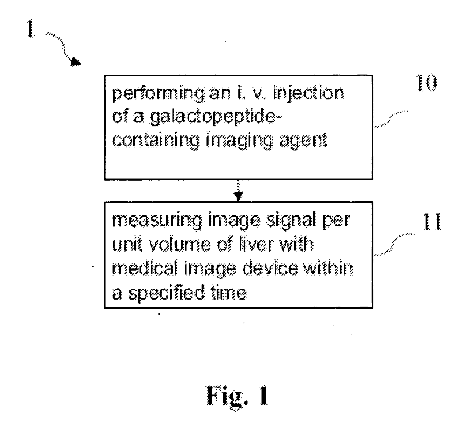

According to Fig 1, method 1 was executed by a glyco-molecular imaging technique for liver targeting. There is no necessity to do any invasive tissue biopsy or body fluids' sampling. The clinical examination steps including step 10 and step 11. In step 10, people were i. v. injected by galactopeptide molecular imaging agent and then the total imaging signal per unit volume of liver was measured by medical imaging device in the step 11. In which, the imaging agent could be radiopharmaceutical or magnetic contrast medium. One example shows radiopharmaceuticals, such as Tc-99m-SOCTA-galactopeptide or Tc-99m-DTPA-galactopeptide, combining SPECT (single positron emission computed tomography) for total Y emitted count per unit volume of liver. Another example shows magnetic contrast medium, such as Mn-DTPA-glactopeptide or Gd-DTPA-galactopeptide, combining MRI (magnetic resonance imaging) for total magnetic resonance signal per unit volume of liver. In steps 11, the measurement time is below 6 hours.

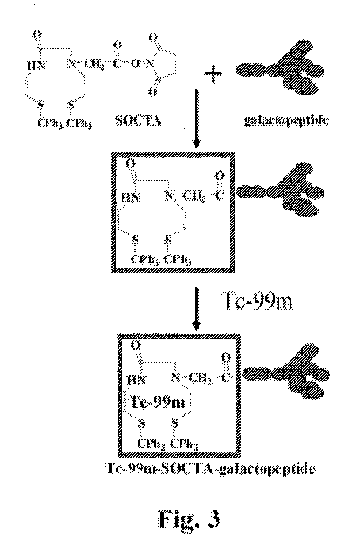

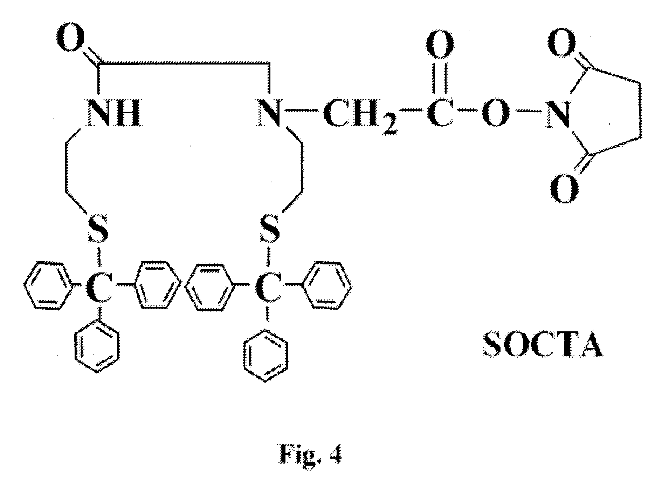

The present invention relates to a galactopeptide imaging agent, such as Tc-99m-SOCTA-galactopeptide, for liver fibrosis diagnosis. The rationale is that the collagen hyperplasia in liver fibrosis will bring the ischemia phenomenon in fibrotic liver. The more collagen produced, the more ischemia existed. Since we know the ischemia will reduce the uptake of imaging agent in liver, we believe Tc-99m-SOCTA-galactopeptide in Fig 3 combining glyco-molecular imaging method for liver targeting could be used for grade differentiation of the fibrotic liver. The molecular imaging is a prior art, so we did not detail here. In Fig 3, SOCTA means (succinimidyl-3,6-diaza-5-oxo-3-[2-((triphenyl-methyl)thio)ethyl]-8-[(triphenylmethyl)-thio]octanoate) and the structure thereof can be referred to in Fig 4.

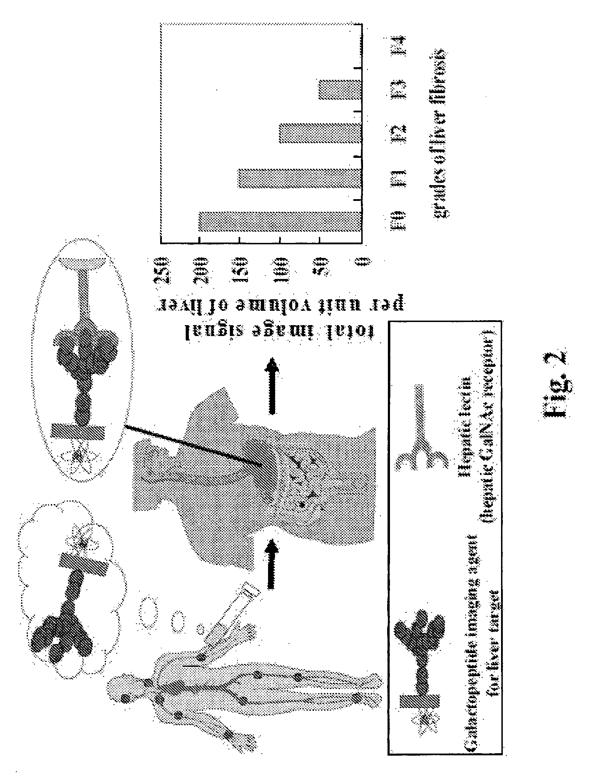

Fig 2 is the graph showing the concept of glyco-molecular imaging for liver targeting. There is no necessity of any liver biopsy or body fluid's sampling. If Tc-99m-SOCTA-galactopeptide or Tc-99m-DTPA-galactopeptide was used as the imaging agent, they will target to liver GalNAc receptor and enter into liver cell by endocytosis after i.v. injection, combining the SPECT (single positron emission computered tomography), the emitted γ ray and liver volume will be measured, then the γ count per unit volume of liver will be measured for liver fibrosis classification. If magnetic contrast medium, such as Mn-D'fPA-galactopeptide or Gd-DTPA-galactopeptide, was used as the imaging agent, they will target to liver GalNAc receptor and enter into liver cell by endocytosis after i.v. injection, combining MRI (magnetic resonance imaging), then the emitted magnetic resonance signal per unit volume of liver will be measured for liver fibrosis classification.

Fig 3 is the preparation flow chart of Tc-99m-SOCTA-galactopetide. The preparation is fulfilled by conjugation of SOCTA with galactopeptide and then labeling with Tc-99m. The conjugation occurs by amino group of galactopeptide reacting with imidazole ester bond of SOCTA. The Tc-99m labeling occurs by linking Tc-99m with N₂S₂ structure of SOCTA. SOCTA could be stored at room temperature lasting for more than one year due to its difficulty of water dissolution and stability at room temperature.

Theoretically, liver fibrosis will cause collagen hyperplasia from the surrounding of portal vein to the surrounding of central vein gradually, so that the blood flow into liver cell, will expectedly reduced. That's why we propose the liver will be under ischemic status. The more collagen hyperplasia, the severity of ischemia. Since prior art indicates "the ischemic hepatic membrane has much lower Gal/GalNAc receptor and meanwhile, the endocytosis is also much less than the normal liver cell.", we propose the fibrotic hepatic membrane has much lower Gal/GalNAc receptor, so that the endocytosis of Tc-99m-SOCTA-galactopeptide will much reduced. That's why we reasonablly derive Tc-99m-SOCTA-galactopeptide combining with the concept of glyco-molecular imaging for liver targeting could be used for liver fibrotic grade classification.

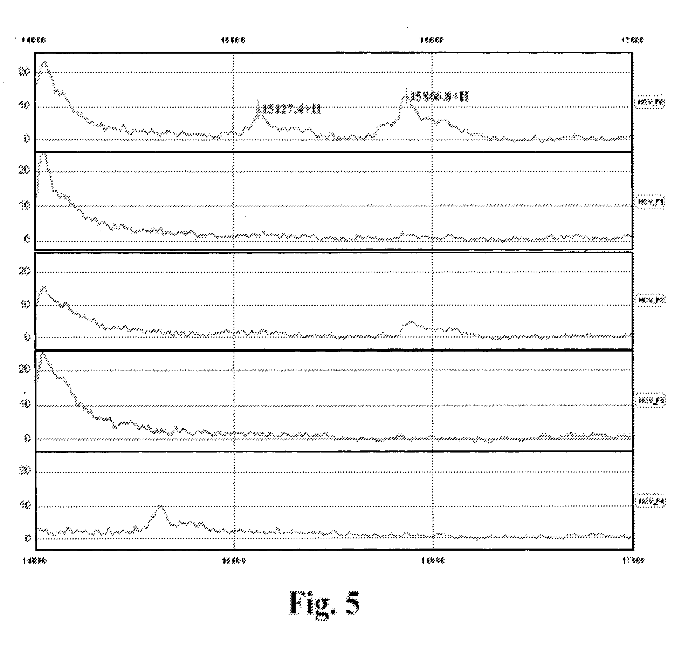

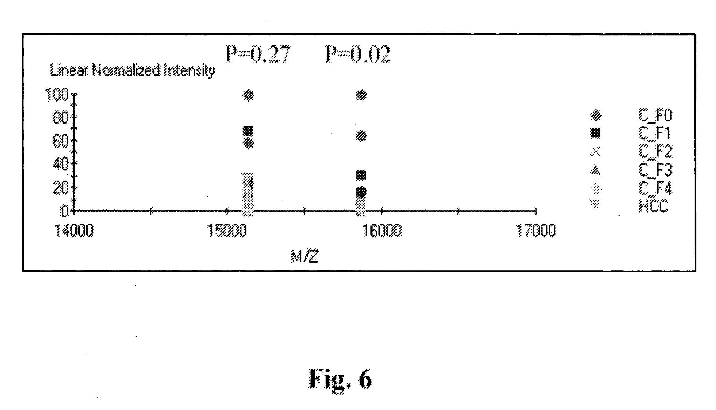

Experimentally, hemoglobin a chain (Molecular weight 15.1kD) and hemoglobin β chain (molecular weight 15.9kD) will reduced under ischemic status. We observe the change of these two protein peaks in serum with SELDI-TOF mass spectrometry. The results indicate the 15.1kD and 15.9kD exactly largely reduced in fibrotic liver. Please see Fig 5 and Fig 6. In fig. 5, the Fo grade of fibrotic liver has higher concentration of these two protein peaks. By statistical analysis of these two protein peaks for 40 liver fibrostic patients in Fig 6, the Fo grade of fibrotic liver has significant difference and higher concentration in 15.9kD (p=0.02) and 15.1kD (p=0.27).

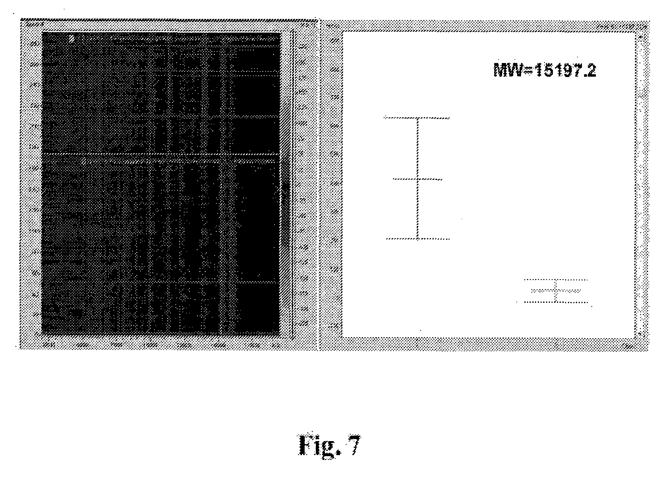

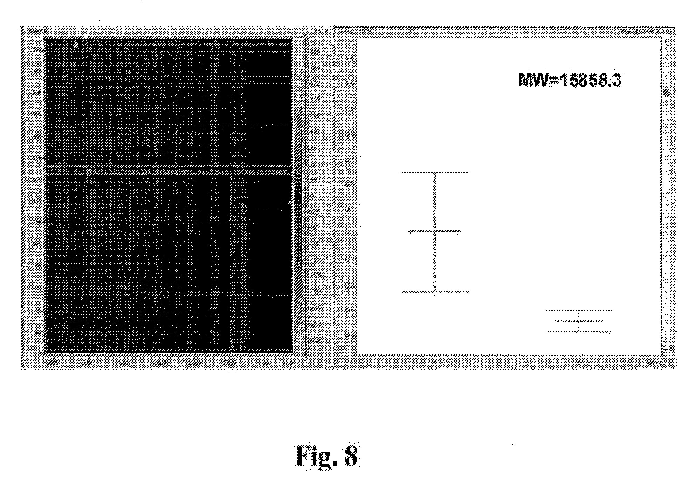

Similar results were shown in tissue profiling. In Fig 7 and 8, sections of 12µ m thickness were obtained on a cryostat at -18 °C and deposited on indium tin oxide coated conductive glass slides. Tissue sections were fixed by immersion in ethanol baths and allowed to dry for 30 min under vacuum. Matrix (for energy absorption in analysis of mass spectrometry) deposition onto the tissue section was performed sequentially with 20mg/mL sinapinic acid dissolved in 50% acetonitrile, 0.1% trifluoroacetic acid. MALDI MS spectra of the spotted tissue sections were acquired on an UltraFlex MALDI-TOF/TOF instrument. Either fig 7 or fig 8 supports fibrotic liver rat was under ischemic status. Fig 7 shows fibrotic liver rat has much less protein expression at 15197.2Da. Fig 8 shows fibrotic liver rat has much less protein expression at 15858.3Da.

Both hemoglobin α chain and β chain reduced in serum and tissue indicate fibrotic liver was under ischemic status. Glyco-molecular imaging for liver targeting method provides image distribution and quantification data. So that if they could be properly labelled with radioisotope or magnetic contrast medium and i. v. injection into body, they will be able to use for grade classification of liver fibrosis and follow-up evaluation of the therapeutic effect.

Liver fibrosis could be totally recovered or improved if early diagnosis of liver fibrosis and administration of proper therapeutical drug. Since glyco-molecular imaging for liver targeting could provide the tissue image distribution and get the quantitative value, it is beneficial to accurate grade classification of fibrotic liver.

A molecular imaging agent, Tc-99m-SOCTA-galactopeptide, was disclosed here which could be used for quantification the severity of fibrotic liver, including grade differentiation. In which, galactopeptide has strong affinity with hepatic GalNAc receptor, and a chelator, such as SOCTA, was essential for linkage between galactopeptide and Tc-99m. Traditionally, DTPA anhydride (DTPA: diethyl-triamine-penta-acetic acid) was used usually. DTPA anhydride reacts pretty quickly (often less than 2 hours) with amino group of galactopeptide in weak alkaline solution, but meanwhile, it is not easy for storage in room temperature because it is much unstable and easy to water-deliquescent. Relatively, SOCTA is much stable and could be stored at room temperature lasting for more than one year. It reacts with galactopeptide much slowly, but it is much stable due to its difficult to water-deliquescent. Its longer shelf-life is an advantage to be an ingredient of imaging agent in a pharmaceutical factory.

Fig 4 is the chemical structure of SOCTA. The imidazole ester bond of SOCTA has the potential to conjugate with the amino group of galactopeptide and the N₂S₂ structure of SOCTA makes it be a good choice for Tc-99m labeling.

Although the present invention has been described with reference to the preferred embodiments, it will be understood that the invention is not limited to the details described thereof.

## Claims

1. Galactopeptide-containing agent for in vivo accurate grade classification of liver fibrosis, which is done with glyco-molecular imaging technique for liver targeting without any necessity for tissue biopsy or body fluid's sampling, comprising measuring imaging signal per unit volume of liver with medical imaging device within a specified time, wherein the galactopeptide-containing imaging agent is Tc-99m-succinimidyl-3,6-diaza-5-oxo-3-[2-((triphenyl-methyl)thio) ethyl]-8-[(triphenylmethyl)-thio]octanoate-glactopeptide and wherein the galactopeptide is trivalent galactose.

2. Galactopeptide-containing agent of claim 1, wherein the Tc-99m-succinimidyl-3,6-diaza-5-oxo-3-[2-((triphenyl-methyl)thio)ethyl]-8-[(triphenylmethyl)-thio] octanoate - -galactopeptide combining single position emission computed tomography is usable for measurement of total γ emitted per unit volume of liver.

3. Galactopeptide-containing agent of claim 1, the specified reaction time of which is below 6 hours.

4. A method for synthesizing glyco-molecular imaging agent with liver targeting for grade classification of liver fibrosis by taking a bifunctional chelator to conjugate galactopeptide and then chelating with a metal, wherein the bifunctional chelator has shelf life lasting for more than one year due to its difficulty for water-deliquescent and good stability at room temperature, wherein the metal is Tc-99m and wherein the bifunctional chelator is succinimidyl-3,6-diaza-5-oxo-3-[2-((triphenyl-methyl)thio) ethyl]-8-[(triphenylmethyl)-thio]octanoate and wherein the galactopeptide is trivalent galactose.

5. The method of claim 4, wherein the imidazole ester bond of succinimidyl-3,6-diaza-5-oxo-3-[2-((triphenyl-methyl)thio) ethyl]-8-[(triphenylmethyl)-thio]octanoate is conjugated with the amino group of galactopeptide.

6. A glyco-molecular imaging agent with liver targeting for grade classification of liver fibrosis being synthesized by taking a bifunctional chelator to conjugate galactopeptide and then chelating with a metal, wherein the bifunctional chelator is succinimidyl-3,6-diaza-5-oxo-3-[2-((triphenylmethyl)thio) ethyl]-8-[(triphenylmethyl)-thio]octanoate and wherein the galactopeptide is trivalent galactose and wherein the metal is Tc-99m, so that the galactopeptide-containing imaging agent is Tc-99m-succinimidyl-3,6-diaza-5-oxo-3-[2-((triphenyl-methyl)thio) ethyl] -8- [(triphenylmethyl)-thio]octanoate-galactopeptide.

## Patentansprüche

1. Galactopeptid - enthaltender Wirkstoff für eine akkurate in-vivo Güteklassen-Einstufung von Leber - Fibrose, welche mittels eines Glyco - Molekular - Bildgebungsverfahrens für Leber - Targeting ohne die Notwendigkeit für Gewebsbiopsie oder Körperflüssigkeitsprobenentnahme durchgeführt wird, welches das Messen eines Bildsignals pro Einheitsvolumen der Leber mit einem medizinischen Bildgebungsgerät innerhalb einer spezifizierten Zeit umfasst, wobei der Galactopeptid - enthaltende Bildgebungswirkstoff Tc-99m-succinimidyl-3,6-diaza-5-oxo-3-[2-((triphenyl-methyl)thio)ethyl]-8-[(triphenylmethyl)-thioloctanoat-galactopeptid ist und wobei das Galactopeptid trivalente Galactose ist.

2. Galactopeptid - enthaltender Wirkstoff für eine akkurate in-vivo Güteklassen-Einstufung von Leber - Fibrose gemäß Anspruch 1, wobei das Tc-99m-succinimidyl-3,6-diaza-5-oxo-3-[2-((triphenyl-methyl)thio)ethyl]-8-[(triphenylmethyl)-thio]octanoat-galactopeptid, das mit der Einzelpositions-emissions-Computer-Tomographie zusammenwirkt, zur Messung der gesamten pro Einheitsvolumen Leber abgestrahlten γ-Strahlung verwendbar ist.

3. Galactopeptid - enthaltender Wirkstoff für eine akkurate in-vivo Güteklassen-Einstufung von Leber - Fibrose nach Anspruch 1, **dadurch gekennzeichnet, dass** die spezifizierte Reaktionszeit weniger als 6 Stunden beträgt.

4. Verfahren zur Synthese des Glyco-Molekular-Bildgebungswirkstoffs mit Leber - Targeting zur Güteklassen-Einstufung von Leber - Fibrose durch Verwendung eines bifunktionalen Chelators zur Konjugation des Galactopeptids und anschließenden Chelatierung mit einem Metall, wobei der bifunktionale Chelator aufgrund der Schwierigkeit zur Wasser - Hygroskopie und der guten Stabilität bei Raumtemperatur eine Haltbarkeitsdauer von mehr als einem Jahr hat, wobei das Metall Tc-99m ist und wobei der bifunktionale Chelator succinimidyl-3,6-diaza-5-oxo-3-[2-((triphenyl-methyl)thio)ethyl]-8-[(triphenylmethyl)-thio]octanoat ist und wobei das Galactopeptid eine trivalente Galactose ist.

5. Verfahren nach Anspruch 4, wobei die Imidazol - Ester - Verbindung des succinimidyl-3,6-diaza-5-oxo-3-[2-((triphenyl-methyl)thio)ethyl]-8-[(triphenylmethyl)-thio]octanoat mit der Amino - Gruppe des Galactopeptid konjugiert ist.

6. Glyco-Molekular-Bildgebungswirkstoff mit Leber - Targeting für eine Güteklassen - Einstufung von Leber - Fibrose, der synthetisiert mittels eines bifunktionalen Chelators, um mit Galactopetid zu konjugieren und mit einem Metall zu chelatieren, wobei der bifunktionale Chelator succinimidyl-3,6-diaza-5-oxo-3-[2-((triphenyl-methyl)thio)ethyl]-8-[(triphenylmethyl)-thio]octanoat ist und wobei das Galactopeptid eine trivalente Galactose ist und wobei das Metall Tc-99m ist, so dass der Galactopeptid - enthaltende Wirkstoff Tc-99m-succinimidyl-3,6-diaza-5-oxo-3-[2-((triphenyl-methyl)thio)ethyl]-8-[(triphenylmethyl)-thio]octanoat - galactopeptid ist.

## Revendications

1. Agent contenant un galactopeptide pour la classification en grade précis in vivo de la fibrose hépatique qui est faite avec une technique d'imagerie glycomoléculaire pour ciblage du foie sans aucune nécessité de biopsie de tissus ou de prélèvement de liquide corporel, comprenant la mesure du signal d'image par unité de volume du foie avec un dispositif d'imagerie médicale dans un délai donné, dans lequel l'agent d'imagerie contenant un galactopeptide est le Tc-99m-succinimidyl-3,6-diaza-5-oxo-3-[2-((triphényl-méthyl)thio)éthyle]-8-[triphénylméthyle)-thio]octanoate-galactopeptide et dans lequel le galactopeptide est le galactose trivalent.

2. Agent contenant un galactopeptide pour la classification en grade précis in vivo de la fibrose hépatique selon la revendication 1 dans lequel la tomographie assistée par ordinateur par émission monophotonique combinant le Tc-99m-succinimidyl-3,6-diaza-5-oxo-3-[2-((triphényl-méthyl)thio)éthyle]-8-[triphénylméthyle)-thio]octanoate-galactopeptide peut être utilisée pour mesurer le Y total émis par unité de volume du foie.

3. Agent contenant un galactopeptide pour la classification en grade précis in vivo de la fibrose hépatique selon la revendication 1, le temps de réaction spécifié duquel est inférieur à 6 heures.

4. Procédé pour synthétiser l'agent d'imagerie glycomoléculaire avec ciblage du foie pour classification en grade de la fibrose hépatique en prenant un chélateur bifonctionnel pour conjuguer le galactopeptide et ensuite le chélater avec un métal, dans lequel le chélateur bifonctionnel a une durée de vie de plus d'un an, ceci étant du à sa difficulté à être déliquescent et à avoir une bonne stabilité à la déliquescence à température ambiante, dans lequel le métal est le Tc-99m et dans lequel le chélateur bifonctionnel est le succinimidyl-3,6-diaza-5-oxo-3-[2-((triphényl-méthyl)thio)éthyle]-8-[triphénylméthyle)-thio]octanoate et dans lequel le galactopeptide est le galactose trivalent.

5. Procédé selon la revendication 4 dans lequel la liaison ester imidazole de succinimidyl-3,6-diaza-5-oxo-3-[2-((triphényl-méthyl)thio)éthyle]-8-[triphénylméthyle)-thio]octanoate est conjuguée au groupe aminé du galactopeptide.

6. Agent d'imagerie glycomoléculaire avec ciblage du foie pour classification en grade de la fibrose hépatique étant synthétisé en prenant un chélateur bifonctionnel pour conjuguer le galactopeptide et ensuite le chélater avec un métal, dans lequel le chélateur bifonctionnel est le succinimidyl-3,6-diaza-5-oxo-3-[2-((triphényl-méthyl)thio)éthyle]-8-[triphénylméthyle)-thio]octanoate et dans lequel le galactopeptide est le galactose trivalent et dans lequel le métal est du Tc-99m si bien que l'agent d'imagerie contenant un galactopeptide est le Tc-99m-succinimidyl-3,6-diaza-5-oxo-3-[2-((triphényl-méthyl)thio)éthyle]-8-[triphénylméthyle)-thio]octanoate-galactopeptide.
